# EUROPÄISCHE PATENTSCHRIFT

(11) **EP 1 071 392 B1**
(45) Veröffentlichungstag und Bekanntmachung des Hinweises auf die Patenterteilung: **03.07.2002**
(21) Anmeldenummer: 99920598.2
(22) Anmeldetag: 06.04.1999
(51) Int. Cl.: A61K 7/09, A61K 7/00

(54) **HAARVERFORMUNGSMITTEL**
HAIR SHAPING AGENTS
AGENT DE MODELAGE CAPILLAIRE

(30) Priorität: 15.04.1998 DE 19816662
(43) Veröffentlichungstag der Anmeldung: 31.01.2001
(73) Patentinhaber: Henkel Kommanditgesellschaft auf Aktien, 40589 Düsseldorf (DE)
(72) Erfinder: FÖRSTER, Thomas, D-40699 Erkrath (DE); PRINZ, Daniela, D-40789 Monheim (DE); HOLLENBROCK, Martina, D-40599 Düsseldorf (DE); MÜLLER, Burkhard, D-21075 Hamburg (DE)
(86) Internationale Anmeldenummer: EP9902324
(87) Internationale Veröffentlichungsnummer: WO9952496

(56) Entgegenhaltungen:
- EP-A- 0 362 663
- WO-A-94/24987
- DE-A- 4 332 805
- US-A- 4 859 459

## Beschreibung

Die Erfindung betrifft ein Verfahren zur Haarverformung durch reduktive Spaltung und erneute oxidative Knüpfung von Disulfidbindungen des Haarkeratins, bei welchem zur Durchführung der keratinreduzierenden Stufe eine Zubereitung in Form einer feinteiligen Öl-in-Wasser-Emulsion zum Einsatz kommt, die eine begrenzte Menge an hydrophilen Emulgatoren enthält.

Unter Haarverformungsmitteln werden im wesentlichen Dauerwellmittel und Haarglättungsmittel verstanden. Kalt-Dauerwellmittel enthalten Reduktionsmittel, welche die Disulfidbindungen des Haarkeratins zu Mercaptogruppen reduzieren. Darüber hinaus können starke Alkalien, z.B. Alkalihydroxide, Ammoniak oder Alkanolamine sowie andere auf Keratin quellend wirkende Stoffe wie z.B. Harnstoff, Guanidin oder Imidazol enthalten sein. Haarglättungsmittel enthalten ebenfalls starke Keratinquellmittel, meist in Kombination mit Keratinreduktionsmitteln. Das Haar kann vor oder nach der Behandlung mit diesen Mitteln mechanisch verformt, z.B. auf Wickler aufgedreht werden. Wenn dann durch eine milde Oxidation ein Teil der Disulfidbrücken des Keratins wieder geknüpft werden, ist die Verformung fixiert.

Die Wirkung des Keratinreduktionsmittels ist jedoch nicht völlig reversibel, sondern hat einige unerwünschte Nebenwirkungen zur Folge. Da nicht alle Disulfidbindungen wiederhergestellt werden, ist die Haltbarkeit der Verformung (curl retention) nicht optimal und die Festigkeit der Haarfaser verringert. Auch die äußere Schuppenschicht des Haars, die sog. Cuticula wird angegriffen, so daß das Haar rauh und schwerer kämmbar wird und einen stumpfen Griff aufweist.

Es bestand daher die Aufgabe, durch geeignete Formulierung der Dauerwellpräparate die unerwünschten Nebenwirkungen möglichst gering zu halten. Dies kann z.B. dadurch erreicht werden, daß man durch Zusatz von Quellmitteln die Haarstruktur lockert und so die aktiven Chemikalien rascher ins Haarinnere eindringen können. Eine weitere Möglichkeit, die Wirksamkeit der aktiven Chemikalien zu erhöhen, besteht in der galenischen Zubereitung, z.B. gemäß WO-A-94/24987 als Mikroemulsion. Dies erfordert aber einen relativ hohen Einsatz von hydrophilen Tensiden, die zur Hautirritation beitragen können.

Es wurde nunmehr gefunden, daß die nach dem Phaseninversionsverfahren erhältlichen, sehr feinteiligen Emulsionen mit mittleren Tröpfchengrößen von weniger als 200 nm, die man mit begrenzten Mengen an hydrophilen Emulgatoren erzeugen kann, als Träger für Haarverformungsmittel überraschend gut geeignet sind, und die Eigenschaften des verformten Haars besonders günstig beeinflussen.

Gegenstand der Erfindung ist daher ein Verfahren zur dauerhaften Haarverformung, bei dem die Haare vor oder nach einer mechanischen Verformung mit einem Keratinreduktionsmittel behandelt, gespült und mit einem Oxidationsmittel fixiert werden, bei dem man zur Keratinreduktion eine feinteilige Öl-in-Wasser-Emulsion mit einem Gehalt von wenigstens 1 Gew.-% eines wasserlöslichen Keratinreduktionsmittels und wenigstens 1 Gew.-% einer emulgierten Öl- oder Fettkomponente mit einer mittleren Tröpfchengröße von weniger als 200 nm und weniger als 0,4 Gewichtsteilen eines hydrophilen Emulgators, bezogen auf 1 Gewichtsteil der Öl- oder Fettkomponente, verwendet.

Als wasserlösliche Keratinreduktionsmittel werden keratinreduzierende Substanzen, ausgewählt aus der Gruppe der Mercaptoverbindungen, Phosphine und der Sulfite eingesetzt. Geeignete Mercaptoverbindungen sind die wasserlöslichen Salze von Mercaptocarbonsäuren mit 2 - 4 C-Atomen wie z.B. der Thioglycolsäure und der Thiomilchsäure, also z.B. Ammoniumthioglycolat, Natrium- oder Kalium-thioglycolat, Monoethanolamin-thioglycolat oder die entsprechenden Thiolactate sowie Cystein und Cysteamin.

Als Sulfite eignen sich bevorzugt die Alkali-, Ammonium- und Alkanolammoniumsalze der schwefligen Säure und der dischwefligen Säure, z.B. Natriumsulfit (Na₂SO₃) oder Natriumdisulfit (Na₂S₂O₅).

Bevorzugt sind als Keratinreduktionsmittel 1 - 20 Gew.-% einer keratinreduzierenden Substanz, ausgewählt aus der Gruppe der Mercaptoverbindungen, der Phosphine oder der Sulfite enthalten.

Ein weiterer Gegenstand der Erfindung ist eine Zubereitung zur Durchführung der keratinreduzierenden Stufe in einem Verfahren zur dauerhaften Haarverformung in Form einer feinteiligen Öl-in-Wasser-Emulsion mit einem Gehalt von wenigstens 1 Gew.-% eines wasserlöslichen Keratinreduktionsmittels und wenigstens 1 Gew.-% einer emulgierten Öl- oder Fettkomponente mit einer mittleren Tröpfchengröße von weniger als 200 nm und weniger als 0,4 Gewichtsteilen eines hydrophilen Emulgators, bezogen auf 1 Gewichtsteil der Öl- oder Fettkomponente.

Als Öl- oder Fettkomponenten werden alle in Wasser unlöslichen Lipide verstanden, bevorzugt jedoch solche, die auch in stark basischen Medien hydrolysestabil sind. Geeignete Öl- oder Fettkomponenten sind z.B. Paraffine (Vaseline), Paraffinöle, Squalan, Fettalkohole (z.B. Cetyl- und Stearylalkohol), 2-Octyldodecanol, Dialkylether usw. Weniger geeignet sind Triglyceride und andere Esteröle.

Besonders bevorzugte Ölkomponenten im Hinblick auf die Stabilität im stark basischen Medium und auf die Herstellung der feinteiligen Emulsionen sind niedermolekulare, flüssige Kohlenwasserstoffe mit 8 - 36 C-Atomen, Dialkylether mit 12 - 36 C-Atomen oder Gemische dieser Komponenten. Geeignete niedermolekulare Kohlenwasserstofe sind vor allem verzweigte Kohlenwasserstoffe oder synthetische Produkte wie z.B. 1,3-Di-(2-ethylhexyl)-cyclohexan, das unter der Bezeichnung Cetiol®S im Handel ist. Geeignete Dialkylether sind z.B. Cetyl-isopropylether, Stearyl-methylether oder symmetrische Dialkylether wie z.B. Di-n-Octylether, der unter der Bezeichnung Cetiol®OE im Handel ist. Die Ölkomponenten sind bevorzugt in Mengen von 5 - 20 Gew.-% enthalten.

Als hydrophile Emulgatoren eignen sich z.B. anionische, zwitterionische, ampholytische oder kationische oberflächenaktive Stoffe, bevorzugt aber nichtionogene Emulgatoren mit einem HLB-Wert oberhalb von 10. Als HLB-Wert soll dabei eine Größe verstanden werden, die sich nach der Emulsionsvergleichsmethode experimentell bestimmen läßt, in grober Näherung läßt sie sich aus der Beziehung HLB = 0,2 (100 - L) abschätzen, worin L der Gewichtsanteil der lipophilen Gruppen, also z.B. der Fettalkyl- oder Fettacyl-Gruppen in Gew.-% im Emulgatormolekül ist. Bevorzugte nichtionische Emulgatoren sind z.B. Fettalkoholpolyglycolether, die durch Anlagerung von mehr als 1 Gewichtsteil Ethylenoxid an 1 Gewichtsteil eines Fettalkohols mit 12 - 22 C-Atomen erhältlich sind. Beispiele solcher Emulgatoren sind z.B. das Anlagerungsprodukt von 10 Mol Ethylenoxid (EO) an Behenylalkohol, das unter der Bezeichnung Mergital®B10 im Handel ist, oder das Anlagerungsprodukt von 12 Mol Ethylenoxid an einen technischen Cetyl/Stearylalkohol-Schnitt, das unter der Bezeichnung Eumulgin®B1 im Handel ist. Bevorzugt eignen sich Fettalkoholpolyglycolether mit HLB-Werten im Bereich von 10 bis 18.

Die erfindungsgemäße Feinteiligkeit der Emulsion läßt sich z.B. nach dem sogenannten PIT-Verfahren dadurch erreichen, daß man bei der Herstellung der Emulsion nichtionische Emulgatoren benutzt und die Emulsion bei der Herstellung über die Phaseninversionstemperatur erwärmt. Das Verfahren ist z.B. in DE 40 10 393 A1 für pola-re Ölkomponenten beschrieben. Für Kohlenwasserstoffe und Dialkylether, die für die erfindungsgemäßen Emulsionen bevorzugt sind, ist die Wahl des HLB-Werts weniger kritisch und auf die Mitverwendung von Coemulgatoren kann gegebenenfalls verzichtet werden.

Ein besonderer Vorteil der erfindungsgemäßen Zubereitungen zur Durchführung der keratinreduzierenden Stufe des Dauerwellverfahrens besteht darin, daß der Emulgatorgehalt sehr niedrig gehalten werden kann und eine Menge von weniger als 0,4 Gewichtsteilen pro Gewichtsteil der Öl- oder Fettkomponenten zur Erreichung der erfindungsgemäßen Feinteiligkeit ausreichend ist.

Nach dem Phaseninversionsverfahren werden in der Regel sehr feinteilige Emulsionen erhalten, deren mittlere Teilchengröße im Bereich von 50 - 200 nm liegt, d.h. es handelt sich noch nicht um klare Mikroemulsionen, deren mittlere Tröpfchengröße unter 50 nm liegt und die frei von Teilchen oberhalb 100 nm sein sollen, um völlig transparent zu erscheinen.

Die typischen, erfindungsgemäß geeigneten PIT-Emulsionen sind opaleszent und haben ein im Auflicht bläuliches, im Durchlicht bräunliches Aussehen. Es kann in besonders günstigen Fällen aber auch schon zur Bildung von klaren oder transparenten Mikroemulsionen kommen.

Aufgrund der hohen Wirksamkeit der erfindungsgemäßen Zubereitungen zur Keratin-Reduktion kann der pH-Wert relativ niedrig, d.h. in Bereichen um den Neutralpunkt bis maximal pH = 9 gehalten werden, ohne daß es zu einer verringerten Haltbarkeit der Verformung kommt. Wie aus den Beispielen ersichtlich, ist bei praktisch gleichem pH-Wert und gleicher Reduktionsmittelkonzentration die Haltbarkeit der Verformung bei Verwendung einer feinteiligen Emulsion deutlich höher als bei einer wäßrigen Lösung.

Auch die Sensorik des verformten Haars, die besonders bei Verwendung von Sulfit oder Disulfit als keratinreduzierende Substanz sehr unbefriedigend ist, wird durch Verwendung der erfindungsgemäßen feinteiligen Emulsion deutlich verbessert. Glanz, Volumen, Kämmbarkeit und Glätte des Haars sind gegenüber einer wäßrigen Lösung des Reduktionsmittels deutlich erhöht. Dies gilt besonders für die Trockenkämmbarkeit des behandelten Haars, die ein Maß für die Oberflächenbeschaffenheit der Cuticula ist.

Neben der emulgierten Öl- oder Fettkomponente, dem Emulgator und dem Keratinreduktionsmittel können in den erfindungsgemäßen Zubereitungen weitere Hilfsstoffe enthalten sein, die entweder die anwendungstechnischen Eigenschaften oder das Aussehen und die sensorischen Eigenschaften der Zubereitungen oder der damit verformten Haare verbessern.

So kann man z.B. die Wirksamkeit der keratinreduzierenden Substanzen durch keratolytische, keratinquellende Stoffe verstärken, die ein rascheres und tieferes Eindringen der reduzierenden Stoffe in die Haarstruktur ermöglichen. Solche Stoffe sind z.B. Harnstoff, Guanidin, Imidazol, organische Amine, z.B. Alkanolamine oder Arginin, Glycolether, Glycerin und niedere Alkohole mit 1 - 4 - C-Atomen. Bevorzugt werden Harnstoff, Imidazol oder Monoethanolamin eingesetzt. In Gegenwart geeigneter Alkalisierungsmittel wie z.B. Alkalihydroxid, Ammoniak oder Ammoniumcarbonat wird die Wirkung der keratinquellenden Komponenten weiter verstärkt.

Die Viskosität der erfindungsgemäßen Zubereitung zur Durchführung der keratinreduzierenden Stufe wird bevorzugt so eingestellt, daß das Mittel leicht auf das mechanisch verformte, z.B. auf Wicklern oder Papilloten befestigte Haar appliziert werden kann und in die Haarsträhnen eindringt, jedoch nicht leicht abtropft oder auf die Kopfhaut läuft. Bevorzugt wird dazu eine Viskosität von 1000 bis 10.000 mPa·s (25° C, D = 10/s) eingestellt.

Ohne besondere Verdickungsmaßnahmen werden bei der Herstellung von Emulsionen nach dem Phaseninversionsverfahren sehr niedrigviskose Emulsionen mit Viskositäten unter 500 mPa·s (25° C, 10 D/s) erhalten. Daher enthalten die erfindungsgemäßen Zubereitungen bevorzugt Verdickungsmittel für die äußere, wäßrige Phase. Als Verdickungsmittel eignen sich alle wasserlöslichen natürlichen und synthetischen polymeren Hydrocolloide, aber auch anorganische Verdickungsmittel, z.B. Schichtsilikate (z.B. Bentonit) oder verdickende Kieselsäuren.

Geeignete natürliche Verdickungsmittel sind z.B. Agar-Agar, Guar-Gum, Johannisbrotkernmehl, Quellstärken und Pektine; synthetische Hydrocolloide sind z.B. die wasserlöslichen Cellulosederivate, z.B. Carboxymethylcellulose oder Hydroxyethylcellulose, vernetzte Polyacrylsäuren, Polyvinylpyrrolidon, Polyvinylalkohol, Polyethylenoxide. Besonders gut eignen sich auch Biopolymere wie Xanthan-Gum oder Gellan-Gum.

Besonders niedrigviskose Zubereitungen eignen sich zur Anwendung als Aerosol-Schaum unter Verwendung geeigneter Treibgase wie z.B. Stickoxydul (N₂O), Dimethylether, Butan oder 1,1,1,2-Tetrafluorethan.

Zur Maskierung des Geruchs werden Duftstoffe oder Geruchskorrigentien eingesetzt, dabei haben alkalistabile, von aldehydischen und hydrolytisch spaltbaren Komponenten freie Parfümöle den Vorzug.

Als weitere Hilfsstoffe können z.B. Trübungsmittel, Farbstoffe, Puffersubstanzen, Komplexbildner und weitere Tenside enthalten sein, deren Gesamtmenge jedoch die Grenze von 0,4 Gewichtsteilen pro Gewichtsteil der Ölkomponente nicht überschreiten sollte.

Schließlich können noch Zusätze zur Verbesserung der Hautverträglichkeit und der haarkosmetischen Eigenschaften, z.B. Aminosäuren, Proteinhydrolysate, kationische Polymere, Silicone, Panthenol, Vitamin oder Pflanzenextrakte enthalten sein.

Das erfindungsgemäße Verfahren verwendet für die weiteren Verfahrensschritte, z.B. für die oxidative Fixierung übliche, für die Dauerwellung bekannte Zubereitungen. Übliche Bestandteile der wäßrigen Fixierlösung sind Oxidationsmittel wie z.B. Wasserstoffperoxid oder Natriumbromat, oberflächenaktive Verbindungen, Stabilisatoren, Duftstoffe und Säuren, z.B. Citronensäure oder Weinsäure zur Einstellung eines pH-Wertes von 3 - 5.

Vor der reduzierenden Stufe des erfindungsgemäßen Verfahrens kann auch noch eine Vorbehandlung unter Verwendung einer Zubereitung mit haarschützenden Komponenten, z.B. mit kationischen Polymeren oder egalisierend wirkenden Komponenten wie z.B. Glucose oder Sorbit durchgeführt werden.

In gleicher Weise kann man nach der Fixierung eine Nachbehandlung des Haars durchführen, die die Quellung wieder verringern und das Haar härter und elastischer machen soll. Solche Nachbehandlungsmittel können z.B. Salze von zwei- oder dreiwertigen Metallen, z.B. Calcium- oder Aluminiumsalze, bevorzugt von Hydroxycarbonsäuren wie z.B. Milchsäure, Citronensäure oder Weinsäure enthalten.

Da die wäßrigen Zubereitungen zur Durchführung des erfindungsgemäßen Verfahrens unmittelbar nacheinander auf das mechanisch verformte und durch Wickler oder Papilloten befestigte Haar angewendet werden, ist es besonders vorteilhaft, wenn die Zubereitungen zur Durchführung des erfindungsgemäßen Verfahrens in den für eine einmalige Anwendung geeigneten und aufeinander abgestimmten Mengen separat verpackt, in einer Verkaufseinheit zusammengefaßt, angeboten werden. Auf diese Weise läßt sich auch für die nichtprofessionelle Heimanwendung des Verfahrens eine ausreichende Sicherheit dafür schaffen, daß der Anwender in Menge und Konzentration optimal aufeinander abgestimmte Zubereitungen zur Keratinreduktion und zur Fixierung verwendet. Eine weitere Variante besteht darin, daß man in die Verkaufseinheit aus Wellmittel und Fixierlösung noch einen separat verpackten Aktivator integriert, der vor der Anwendung des Wellmittels (Keratinreduktionsmittel) mit diesem vereinigt wird und zu einer Temperaturerhöhung im Keratinreduktionsmittel führt. Auf diese Weise kann die Wirkung des Wellmittels gesteigert und die erforderliche Einwirkungszeit auf das Haar abgekürzt werden. Als Aktivator kann dabei eine Substanz dienen, die mit den Komponenten des Keratinreduktionsmittels z.B. durch Hydratation mit dem Wasser oder durch Oxidation eines Teils des Reduktionsmittels Wärme entwickelt. Solche geeigneten Aktivatoren sind z.B. Wasserstoffperoxid oder dessen Additionsprodukte, wasserfreie Zeolithe oder wasserfreie Salze mit positiver Lösungswärme, z.B. Magnesiumsulfat. Durch eine beigepackte Gebrauchsanleitung kann der Verwender Fehldosierung und Falschanwendung vermeiden.

Die folgenden Beispiele sollen den Gegenstand der Erfindung weiter erläutern:

### Beispiele

Es wurden erfindungsgemäße Zubereitungen zur Durchführung der keratinreduzierenden Stufe eines Dauerwellverfahrens hergestellt.

**Tabelle 1**

| **Beispiel** | **1** | **2** | **V** |
|---|---|---|---|
| Cetiol®OE | 5,7 | 5,7 | - |
| Cetiol®S | 5,7 | 5,7 | - |
| Mergital®B10 | 3,0 | 3,0 | - |
| Structure®3001 (30 %ig) | 3,0 | 3,0 | - |
| HS-CH₂-COONH₄ (50%ig in Wasser) | 20,0 | - | - |
| Na₂S₂O₅ | - | 6,9 | 6,9 |
| Harnstoff (50 Gew.-% in Wasser) | - | 30,6 | 30,6 |
| NH₄HCO₃ | 7,05 | - | - |
| Ammoniak-Lösung (25 %ig) | 1,68 | 6,3 | 3,1 |
| Turpinal®SL (60 %ig) | 0,50 | - | - |
| Texapon®NSO (28 %ig) | - | 2,7 | 2,7 |
| Wasser | ad 100 | ad 100 | ad 100 |
| pH - Wert | 8,3 | 7,3 | 6,5 |
| Trockenkämmbarkeit [mJ] | - | 196 | 625 |
| Haltbarkeit (%) | 81 | 95 | 89 |

### Herstellungsweise der erfindungsgemäßen Produkte 1 und 2

Aus den Ölkomponenten (Cetiol OE, Cetiol S) und dem Emulgator (Mergital B10) sowie 10 Gewichtsteilen Wasser wurde eine feinteilige Emulsion nach dem PIT-Verfahren hergestellt. Nach Abkühlung auf 40°C wurde das Verdickungsmittel zugesetzt. Die übrigen Komponenten wurden zu einer wäßrigen Lösung vereinigt und mit der Emulsion gemischt.

### Herstellung des Vergleichsprodukts V

Die Komponenten wurden zu einer wäßrigen Lösung vereinigt.

Es wurden folgende Handelsprodukte verwendet:

| | |
|---|---|
| Cetiol®OE | Di-n-octylether |
| Cetiol®S | 1,3-Bis-(2-ethylhexyl)-cyclohexan |
| Mergital®B10 | Behenylalkoholpoly-(10EO)-glycolether |
| Structure®3001 | Acrylates/Ceteth-20-Itaconate Copolymer |
| Natrosol®250 HR | Hydroxyethylcellulose |
| Texapon®NSO | Fettalkohol(C₁₂₋₁₄)-poly(2EO)glycolether-sulfat, Na |
| Turpinal®SL | 1-Hydroxyethan-1,1-diphosphonsäure |

### Anwendungstechnische Prüfung

### 1. Haltbarkeit (curl retention)

Haarsträhnen von 27 cm Länge und 0,6 g Gewicht wurden auf einem Standard-Wellbrett in Zickzackwellen gezwungen und in dieser Lage mit dem Dauerwellmittel behandelt, gespült, mit einer Fixierlösung behandelt und erneut gespült. Als Dauerwellmittel wurden die Zusammensetzungen der Beispiele 1 und 2 sowie der Vergleichsprodukte 1V und 2V gewählt, als Fixierlösung diente eine wäßrige Lösung von 3,5 Gew.-% Kaliumbromat, 7 Gew.-% Fettalkoholpolyglycolethersulfat-Na-Salz, 5 Gew.-% Kokosfettsäurediethanolamid, 0,15 Gew.-% Weinsäure, 0,3 Gew.-% Parfümöl und 1 Gew.-% Natriumchlorid.

Nach dem Trocknen wurde die Strähne aus dem Wellbrett genommen. Die Länge von 6 Amplituden der Welle definiert den Wellwert. Dann wurde die Haarsträhne mit einem Gewicht von 50 g belastet und senkrecht 60 Minuten in einen mit Wasser gefüllten Zylinder gehängt. Nach der Entlastung wurde wieder die Länge von 6 Amplituden gemessen. Die Haltbarkeit (curl retention) ergibt sich aus der Beziehung Cr(%) = 100 · (l-l₆₀)/(l-lₒ). Dabei bedeuten:
- l =: Länge der 6 Amplituden im strammgezogenen Zustand
- lₒ =: Länge nach Dauerwellbehandlung
- l₆₀ =: Länge nach 60 Minuten Belastung.

### 2. Trockenkämmbarkeit

Zwanzig Haarsträhnen (je 15 cm lang, 2 g schwer) wurden mit Dauerwell-Lösung behandelt, gespült, fixiert, gespült und getrocknet, ohne eine mechanische Verformung der Haare vorzunehmen. Dann wurden die Strähnen bei 20 % relativer Luftfeuchte und 30° C für 12 Stunden gelagert (konditioniert) und dann gekämmt. Dabei wurden 10 Kämmstriche in einer vollautomatischen Apparatur ausgeführt und die aufgewendete Kämmarbeit gemessen.

Der Trockenkämmbarkeits-Wert [in mJ] ist das mittlere Arbeitsintegral über alle 20 Probesträhnen.

## Patentansprüche

1. Verfahren zur dauerhaften Haarverformung, bei dem die Haare vor und/oder nach einer mechanischen Verformung mit einem Keratinreduktionsmittel behandelt, gespült und mit einem Oxidationsmittel fixiert werden, **dadurch gekennzeichnet, daß** man zur Keratinreduktion eine feinteilige Öl-in-Wasser-Emulsion mit einem Gehalt von wenigstens 1 Gew.-% eines wasserlöslichen Keratinreduktionsmittels und wenigstens 1 Gew.-% einer emulgierten Öl- oder Fettkomponente mit einer mittleren Tröpfchengröße von weniger als 200 nm und weniger als 0,4 Gewichtsteilen eines hydrophilen Emulgators, bezogen auf 1 Gewichtsteil der Öl- oder Fettkomponente, verwendet.

2. Verfahren nach Anspruch 1, **dadurch gekennzeichnet, daß** als wasserlösliches Keratinreduktionsmittel 1 - 20 Gew.-% einer keratinreduzierenden Substanz, ausgewählt aus der Gruppe der Mercaptoverbindungen der tert. Phosphine und der Sulfite, enthalten ist.

3. Zubereitung zur Durchführung der keratinreduzierenden Stufe in einem Verfahren zur dauerhaften Haarverformung in Form einer feinteiligen Öl-in-Wasser-Emulsion mit einem Gehalt von wenigstens 1 Gew.-% eines wasserlöslichen Keratinreduktionsmittels und wenigstens 1 Gew.-% einer emulgierten Öl- oder Fettkomponente mit einer mittleren Tröpfchengröße von weniger als 200 nm und weniger als 0,4 Gewichtsteilen eines hydrophilen Emulgators, bezogen auf 1 Gewichtsteil der Öl- oder Fettkomponente.

4. Zubereitung nach Anspruch 3, **dadurch gekennzeichnet, daß** als emulgierte Öl- oder Fettkomponente wenigstens ein flüssiger Kohlenwasserstoff mit insgesamt 8 bis 36 C-Atomen oder ein Dialkylether mit insgesamt 12 - 36 C-Atomen oder ein Gemisch davon in einer Menge von 5 - 20 Gew.-% enthalten ist.

5. Zubereitung nach einem der Ansprüche 3 oder 4, **dadurch gekennzeichnet, daß** als Keratinreduktionsmittel ein Alkali- oder Ammoniumsalz der Thioglycolsäure, der Thiomilchsäure oder der schwefligen Säure enthalten ist.

6. Zubereitung nach einem der Ansprüche 3 - 5, **dadurch gekennzeichnet, daß** als Keratinreduktionsmittel ein Sulfit und zusätzlich eine keratinquellende Substanz, ausgewählt aus Harnstoff, Imidazol und Monoethanolamin enthalten ist.

7. Zubereitung nach einem der Ansprüche 3 - 6, **dadurch gekennzeichnet, daß** zusätzlich Verdickungsmittel für die äußere, wäßrige Phase enthalten sind.

## Claims

1. A process for permanently shaping hair in which, before or after mechanical shaping, the hair is treated with a keratin reducing agent, rinsed and fixed with an oxidizing agent, **characterized in that** a fine-droplet oil-in-water emulsion containing at least 1 % by weight of a water-soluble keratin reducing agent and at least 1% by weight of an emulsified oil or fatty component with a mean droplet size of less than 200 nm and less than 0.4 part by weight of a hydrophilic emulsifier, based on 1 part by weight of the oil or fatty component, is used for keratin reduction.

2. A process as claimed in claim 1, **characterized in that** 1 to 20% by weight of a keratin reducing chemical selected from the group of mercapto compounds, tert. phosphines and sulfites is present as the keratin reducing agent.

3. A preparation for carrying out the keratin reducing step of a process for the permanent shaping of hair in the form of a fine-droplet oil-in-water emulsion containing at least 1% by weight of a water-soluble keratin reducing agent and at least 1% by weight of an emulsified oil or fatty component with a mean droplet size of less than 200 nm and less than 0.4 part by weight of a hydrophilic emulsifier, based on 1 part by weight of the oil or fatty component.

4. A preparation as claimed in claim 3, **characterized in that** at least one liquid hydrocarbon containing a total of 8 to 36 carbon atoms or a dialkyl ether containing a total of 12 to 36 carbon atoms or a mixture thereof is present in a quantity of 5 to 20% by weight as the emulsified oil or fatty component.

5. A preparation as claimed in claim 3 or 4, **characterized in that** an alkali metal or ammonium salt of thioglycolic acid, thiolactic acid or sulfurous acid is present as the keratin reducing agent.

6. A preparation as claimed in any of claims 3 to 5, **characterized in that** a sulfite and in addition a keratin swelling chemical selected from urea, imidazole and monoethanolamine are present as the keratin reducing agent.

7. A preparation as claimed in any of claims 3 to 6, **characterized in that** thickeners for the outer aqueous phase are additionally present.

## Revendications

1. Procédé pour la déformation permanente des cheveux, dans lequel les cheveux sont traités par un réducteur de la kératine, avant et/ou après une déformation mécanique, rincés et fixés à l'aide d'un oxydant,
**caractérisé en ce qu'**
on utilise pour la réduction de la kératine une émulsion huile-dans-eau finement divisée, contenant au moins 1 % en poids d'un réducteur de kératine soluble dans l'eau et au moins 1 % en poids d'un composant de type huile ou graisse émulsionné, ayant une taille moyenne de gouttelettes de moins de 200 nm, et moins de 0,4 partie en poids d'un émulsifiant hydrophile, par rapport à 1 partie en poids du composant de type huile ou graisse.

2. Procédé selon la revendication 1,
**caractérisé en ce que**
comme réducteur de kératine soluble dans l'eau, sont contenus 1-20 % en poids d'une substance réduisant la kératine, choisie dans le groupe des composés mercapto, des phosphines tertiaires et des sulfites.

3. Préparation pour l'exécution de l'étape de réduction de la kératine dans un procédé pour la déformation permanente des cheveux, sous forme d'une émulsion huile-dans-eau finement divisée, contenant au moins 1 % en poids d'un réducteur de kératine soluble dans l'eau et au moins 1 % en poids d'un composant de type huile ou graisse émulsionné, ayant une taille moyenne de gouttelettes de moins de 200 nm, et moins de 0,4 partie en poids d'un émulsifiant hydrophile, par rapport à 1 partie en poids du composant de type graisse ou huile.

4. Préparation selon la revendication 3,
**caractérisée en ce qu'**
elle contient comme composant de type huile ou graisse émulsionné au moins un hydrocarbure liquide ayant au total de 8 à 36 atomes de carbone, ou un éther dialkylique ayant au total de 12 à 36 atomes de carbone, ou un mélange de ceux-ci, en une quantité de 5-20 % en poids.

5. Préparation selon la revendication 3 ou 4,
**caractérisée en ce qu'**
elle contient en tant que réducteur de la kératine un sel de métal alcalin ou d'ammonium de l'acide thioglycolique, de l'acide thiolactique ou de l'acide sulfureux.

6. Préparation selon l'une quelconque des revendications 3 à 5,
**caractérisée en ce qu'**
elle contient comme réducteur de la kératine un sulfite et en outre une substance faisant gonfler la kératine, choisie parmi l'urée, l'imidazole et la monoéthanolamine.

7. Préparation selon l'une quelconque des revendications 3 à 6,
**caractérisée en ce qu'**
elle contient en outre des épaississants pour la phase aqueuse externe.
